# EUROPÄISCHE PATENTSCHRIFT

(11) **EP 2 322 079 B1**
(45) Veröffentlichungstag und Bekanntmachung des Hinweises auf die Patenterteilung: **22.04.2015**
(21) Anmeldenummer: 10190378.9
(22) Anmeldetag: 08.11.2010
(51) Int. Cl.: A61B 1/00, A61B 1/005

(54) **Endoskopschaft aus einem Verbundschlauch**
Endoscopic shaft from a composite hose
Arbre d'endoscope à partir d'un tuyau composite

(30) Priorität: 11.11.2009 DE 102009052688
(43) Veröffentlichungstag der Anmeldung: 18.05.2011
(73) Patentinhaber: invendo medical GmbH, 86438 Kissing (DE)
(72) Erfinder: Dillinger, Ilona Sabine, 86447 Aindling (DE); Großhardt, Manfred Josef, 86343 Königsbrunn (DE)
(74) Vertreter: Winter, Brandl, Fürniss, Hübner, Röss, Kaiser, Polte - Partnerschaft mbB

(56) Entgegenhaltungen:
- EP-A1- 0 913 165
- WO-A1-94/15522
- WO-A2-2005/068887
- DE-B3- 10 359 719
- DE-U1- 8 807 415
- DE-U1-202005 004 169
- JP-A- 6 189 898
- US-A1- 2007 255 105
- US-A1- 2008 139 887

## Beschreibung

Die vorliegende Erfindung betrifft einen Endoskopschaft aus einem Verbundschlauch gemäß dem Oberbegriff des Patentanspruchs 1 und wie im nächstliegenden Stand der Technik dargelegt, der durch die Schriften WO94/15522 und US 2008/139887 repräsentiert wird.

Endoskope werden in jüngster Zeit über eine erhebliche Strecke von bis zu 2,5 m beispielsweise in den Kolon eines Patienten eingeführt, um diesen auf krankhafte Gewebeveränderungen, wie beispielsweise Krebs, Polypen, Analfisteln und dergleichen zu untersuchen und gegebenenfalls auch zu behandeln. Zur Überwindung dieser extrem langen Einführstrecke sind neuartige Endoskope mit Antriebsvorrichtungen, beispielsweise bestehend aus einem den Endoskopschaft umgebenden Stülpschlauch ausgerüstet, der wiederum mittels einer Antriebsvorrichtung vorwärts bewegbar ist und dabei eine Vorschubkraft auf den Endoskopschaft ausübt.

Aus dem Stand der Technik, insbesondere der Anmelderin selbst, ist ein derartiges Endoskop mit einem Stülpschlauchantrieb für das Vorwärts- und Rückwärtstreiben eines Endoskopschafts im Rahmen einer Vielzahl von Patentveröffentlichungen bekannt. Ein derartiges Endoskop besteht im Wesentlichen aus einem Endoskopschaft, der in einem Stülpschlauch gleitend eingesetzt ist. Der Stülpschlauch wird hierbei aus einem an einem distalen sowie proximalen Ende des Endoskopschafts umgestülpten Schlauch aus PVC oder Silikon gebildet (Doppelstülpschlauchkonstruktion), in dessen Mittenabschnitt eine Antriebsvorrichtung angeordnet ist. Die jeweils nach außen umgestülpten Enden des Schlauchs sind zu dieser Antriebsvorrichtung zurückgeführt und vorzugsweise am Gehäuse der Antriebsvorrichtung fixiert.

Die Antriebsvorrichtung selbst, besteht entweder aus einem kontinuierlichen Vortriebsmechanismus vorzugsweise aus einer Anzahl von Antriebsrädern oder einer Raupe oder aus einem diskontinuierlichen Antriebsmechanismus. Grundsätzlich treibt die Antriebsvorrichtung lediglich den Stülpschlauch an, welcher wiederum eine Antriebskraft auf den Endoskopschaft aufbringt. Hierfür wird die Antriebsvorrichtung beziehungsweise deren Antriebsmechanismus in Reibkontakt mit dem inneren Stülpschlauchabschnitt gebracht und reibschlüssig eine Vorschubkraft auf diesen übertragen. Diese Vorschubkraft auf den inneren Abschnitt des Stülpschlauchs bewirkt eine entsprechende Vorwärtsbewegung des distalen Umstülpabschnitts, welches sich an einem am Endoskopschaft fixierten Anschlagstück anlegt und dort abgleitet. Diese Vorwärtsbewegung des distalen Umstülpabschnitts des Stülpschlauchs wird demzufolge über das Anschlagstück auf den Endoskopschaft übertragen, wodurch dieser innerhalb des inneren Stülpschlauchabschnitts mitgezogen wird.

Aufgrund der kinematischen Bedingungen ist demzufolge die Vortriebsgeschwindigkeit des inneren Stülpschlauchabschnitts doppelt so groß wie die Vortriebsgeschwindigkeit des Endoskopschafts, sodass eine relative Gleitbewegung als Bewegungssynchronisation erforderlich ist.

Aus der vorstehenden Beschreibung des Stands der Technik wird ersichtlich, dass für das Übertragen einer ausreichenden Vortriebskraft auf den inneren Stülpschlauchabschnitt der Antriebsmechanismus mit einer vorbestimmten Minimumsaufstandskraft gegen den inneren Stülpschlauchabschnitt gepresst werden muss. Diese bewirkt jedoch ein im Wesentlichen punktuelles Eindrücken des Endoskopschafts im Bereich des Antriebsmechanismus. Darüber hinaus muss an dieser Stelle eine höhere Reibung zwischen Endoskopschaft und innerem Stülpschlauchabschnitt überwunden werden, was nur durch eine ausreichende Schmierung des Endoskopschafts überhaupt ermöglicht wird. Des Weiteren wurde festgestellt, dass der Antriebsmechanismus den Endoskopschaft kurzfristig oder langzeitig deformiert, was zu einem ungleichmäßigen Vorschub des Stülpschlauchs und des darin gelagerten Endoskopschaft führt.

Um das Endoskop überhaupt um die vorstehend bezeichnete Strecke in den Kolon eines Patienten einführen zu können, ist es erforderlich, dass der Endoskopschaft eine hohe Flexibilität und Biegeweichheit aufweist. Dies steht jedoch der notwendigen Eigenschaft des Endoskopschafts gegenüber, ein ausreichend steifes Widerlager für den Antriebsmechanismus zu bilden.

Angesichts dieser Problematik ist es die Aufgabe der vorliegenden Erfindung, einen Endoskopschaft aus einem Verbundmaterial bereit zu stellen, welcher eine ausreichende Biegeflexibilität aufweist, als Widerlager für die Antriebsvorrichtung eines den Endoskopschaft umgebenden Stülpschlauchs einsetzbar ist und darüber hinaus zu einer Verringerung der Reibung zwischen dem Endoskopschaft und diesen umgebenden Stülpschlauch beiträgt.

Dieser Aufgabe wird erfindungsgemäß mittels eines Endoskopschafts aus einem Verbundmaterial mit den Merkmalen des Patentanspruchs 1 gelöst.

Vorteilhafte Weiterbildungen der Erfindung sind dabei Gegenstand der Unteransprüche.

Der Kern der Erfindung besteht demzufolge darin, den Endoskopschaft aus einem Verbundschlauch mit einem Außenschlauch und einem zum Außenschlauch weicheren und/oder biegeflexibleren Innenschlauch sowie einer Spiralfeder zur Aussteifung des Verbundschlauchs zu fertigen, die erfindungsgemäß in entspanntem Zustand einen Außendurchmesser hat, der (geringfügig) größer ist als der Innendurchmesser des Innenschlauchs. Die Spiralfeder ist dabei in den Innenschlauch vorzugsweise lose unter einer bestimmten Federvorspannung (in Abhängigkeit der Federsteifigkeit sowie des radialen Übermaßes) eingesetzt, wodurch sie sich gegen den Innenschlauch radial andrückt.

Die Spiralfeder wirkt somit nicht nur aufgrund ihrer Materialsteifigkeit sondern auch infolge ihres Einbaus unter radialer Vorspannung als verbessertes Widerlager für eine nachträglich angeordnete Antriebsvorrichtung für den Endoskopschaft und verhindert so nachhaltig radiale Deformierungen des Endoskopschafts infolge der Anpresskräfte der Antriebsvorrichtung.

Vorzugsweise ist die radial nach Außen gerichtete Andrückkraft der Spiralfeder auf den Innenschlauch so groß gewählt, dass die Spiralfeder durch die Wandungen des Innenschlauchs und Außenschlauchs hindurch eine Struktur auf der Außenseite des Außenschlauchs aufprägt. Weiter vorzugsweise entspricht die Struktur auf der Außenseite des Außenschlauchs den Federwindungen der Spiralfeder. Dies hat den Vorteil, dass ein Stülpschlauch, der als Antrieb um den Endoskopschaft herum relativ verschieblich angeordnet ist und auf den die Antriebskraft der Antriebsvorrichtung einwirkt, eine verringerte Kontaktfläche mit dem Endoskopschaft hat, sodass die Reibung zwischen Endoskopschaft und Stülpschlauch reduziert werden kann. Des Weiteren bildet die sich abzeichnende Außenseitenstruktur (Oberflächenstruktur) Aufnahmevertiefungen (Taschen) aus, in denen sich eingepresstes Schmiermittel sammelt und dadurch weiter die Reibung verringert.

Vorteilhaft kann es hierbei sein, die Spiralfeder über deren Axiallänge mit unterschiedlichen Federsteifigkeiten und/oder Steigungen und/oder Außendurchmessern auszubilden, um so die abschnittsweise Biegeflexibilität des Endoskopschafts über dessen Länge von vorzugsweise 2.5 m zu optimieren.

Die Erfindung schafft demzufolge auch ein Endoskop mit einem solchen Stülpschlauchantrieb bestehend aus einem vorzugsweise doppelt gestülpten Antriebsschlauch, der von einer Antriebseinrichtung umgriffen ist. Die Antriebsvorrichtung hat Antriebsmittels (beispielsweise Reibräder), die in Reibeingriff mit dem Antriebsschlauch stehen, um einer Vorschubkraft auf den Antriebsschlauch aufzubringen. Erfindungsgemäß ist ein Endoskopschaft mit den vorstehenden Merkmalen vorgesehen, der in dem Antriebsschlauch gleitend geführt ist und der das radiale Widerlager für die Antriebsmittel bildet.

Die Erfindung wird nachstehend anhand eines bevorzugten Ausführungsbeispiels unter Bezugnahme auf die einzige begleitende Figur näher erläutert. Diese Figur zeigt die Seitenansicht eines Endoskopschafts für ein erfindungsgemäßes Endoskop mit Stülpschlauchantrieb gemäß dem bevorzugten Ausführungsbeispiel der Erfindung.

Demzufolge besteht der Endoskopschaft aus einem Verbundschlauch 1 mit einer sogenannten Reibungsoptimierten Außenfläche. Im Konkreten ist der Verbundschlauch aus zwei co-extrodierten oder miteinander verklebten Schläuchen aufgebaut bestehend aus einem inneren Schlauch und einem äußeren Schlauch, wobei der innere Schlauch aus einem weicheren Material gefertigt ist als der äußere Schlauch. Der innere Schlauch gewährleistet eine Minimumsflexibilität des Verbundschlauchs wohingegen der Außenschlauch eine Minimunsdeformationsfestigkeit sowie gute Reibeigenschaften an dessen Oberfläche gewährleistet. Im Konkreten weist der Innenschlauch eine Shore-Härte von ca. 60 Shore A und der Außenschlauch eine Shore-Härte von ca. 92 Shore A auf. Der Innenschlauch ist ferner vorzugsweise aus einem PVC-Material und der Außenschlauch aus einem PUR-Material gefertigt.

In den Verbundschlauch 1 ist eine Spiralfeder 2 zur radialen Aussteifung eingeklemmt (eingeblasen).

Im Konkreten hat der innere Schlauch einen Innendurchmesser, der geringfügig kleiner ist, als der Außendurchmesser einer Spiralfeder 2 in entspanntem Zustand. Diese Feder 2 wird für den Einblasvorgang tordiert, wodurch sich deren Durchmesser verkleinert. Daraufhin wird die Feder in den Innendurchmesser des inneren Schlauches über dessen im Wesentlichen gesamte Länge eingeschoben und entspannt. Hierdurch weitet sich der Durchmesser der Spiralfeder 2 auf, wodurch diese eine Radialkraft auf den Verbundschlauch 1 ausübt, die zu einer geringfügigen Aufweitung des Verbundschlauchs 1 führt.

Im Konkreten bewirkt die radiale Aufspannkraft der eingezogenen Spiralfeder 2 ein Durchdrücken der einzelnen Federwindungen bis zur Außenseite des Verbundschlauchs 1, wodurch sich dort ein spiralförmiges Rippenmuster mit geringfügigen radialen Auswölbungen und Vertiefungen abzeichnet. Die Federgeometrie selbst ist vorzugsweise rechtwinklig ausgestaltet, um ein Abkippen der lediglich eingeschobenen Feder 2 beispielsweise durch Rückstellkräfte des Verbundschlauchs 1 oder durch die Anpresskräfte einer Antriebsvorrichtung des mit diesem Endoskopschaft ausgerüsteten Endoskops weitestgehend auszuschließen. Darüber hinaus wird durch die rechtwinklige Federgeometrie der freie Innendurchmesser des Verbundschlauchs (= Verbundschlauch und Flachfeder zusammen) vergrößert.

Am proximalen Ende des Verbundschlauchs 1, d. h. an jenem hinteren Ende, das im Betrieb des fertig gestellten Endoskops aus der Körperöffnung eines Patienten herausragt, ist eine Schmiertülle 4 aus einem Metallstück angeordnet, auf die der Verbundschlauch 1 sowie die Flachfeder 3 gemeinsam festgeklemmt (gekrimmt) sind.

Alternativ zu der vorstehend beschriebenen rechwinkligen Federgeometrie ist es auch möglich, die Feder kreis- oder ovalförmig auszugestalten. Des Weiteren besteht optional die Möglichkeit, eine Feder mit zwei in deren Federlängsrichtung unterschiedlichem Steigungen einzusetzen. Hierdurch wird die Steifigkeit des Verbundschlauchs 1 als Funktion von dessen Länge beeinflusst. Dies kann vorteilhaft für die Optimierung des "Fahrwegs" innerhalb des Kolon eines Patienten sein.

Eine weitere Möglichkeit der Optimierung der verwendeten Spiralfeder besteht darin, den Außendurchmesser der Feder in über deren Längsrichtung regelmäßig beabstandeten Sprüngen zu variieren. Dem liegt die Beobachtung zu Grunde, dass die Spiralfeder eine gewisse Struktur auf der Außenseite des Verbundschlauchs gemäß vorstehender Beschreibung aufträgt, die demzufolge unterschiedlich stark in Längsrichtung des Verbundschlauches ausgebildet sein kann. Die Vorteile sowie Auswirkungen der erfindungsgemäßen Endoskopschaftkonstruktion auf ein mit diesem ausgerüsteten Endoskop mit Stülpschlauchantrieb lässt sich wie folgt zusammenfassen:

Wie eingangs bereits ausgeführt wurde, hat ein mit dem beschriebenen Endoskopschaft ausgerüstetes Endoskop einen Stülpschlauchantrieb bestehend aus einem vorzugsweise doppelt gestülpten Schlauch, der von einer Antriebseinrichtung für eine reibschlüssige Vortriebskraftübertragung umgriffen wird. Diese Antriebseinrichtung bringt hierbei Antriebsmittel beispielsweise Antriebsräder oder eine Raupe in Reibkontakt mit einem radial inneren Schlauchabschnitt, um diesen in Längsrichtung des Stülpschlauchs anzutreiben. Diese Antriebsbewegung des inneren Stülpschlauchabschnitts wird an den vorderen (distalen) und/oder hinteren (proximalen) Umstülpbereichen des Stülpschlauchs auf den innerhalb des Stülpschlauchs relativ verschiebbar gelagerten Endoskopschaft übertragen. Aus den eingangs genannten Gründen ist hierbei diese relative Verschiebbarkeit zwischen dem Endoskopschaft und dem inneren Schlauchabschnitt des Antriebsschlauchs von essentieller Bedeutung.

Die Antriebsmittel müssen für einen ausreichenden Reibschluss mit einer bestimmten radialen Kraft auf dem inneren Stülpschlauchabschnitt gepresst werden, wobei hierfür der innen liegende Endoskopschaft als Widerlager für die Antriebsmittel dient. Es liegt auf der Hand, dass an dieser Stelle die Reibung zwischen dem Endoskopschaft und dem inneren Stülpschlauchabschnitt zunimmt.

Die Verwendung von zwei auf deren Funktion optimierten Materialien trägt dieser Situation Rechnung. Das heißt, der weiche, biegeflexible Innenschlauch gewährleistet eine Vorwärtsbewegung des Endoskopschafts in den Kolon eines Patienten, indem der Endoskopschaft biegeflexibel den Krümmungen des Kolons folgt, ohne diesen über Gebühr aufzuweiten. Der gegenüber dem inneren Endoskopschaftsschlauch härtere Außenschlauch gewährleistet eine vorbestimmte minimale Steifigkeit in radialer Richtung und dient so als Widerlager für die Antriebsmittel eines Stülpschlauchantriebs. Es hat sich hierbei gezeigt, dass der Außenschlauch eine wesentlich geringere Wandstärke aufweisen kann als der Innenschlauch und trotzdem eine ausreichende radiale Steifigkeit behält.

Die in den inneren Schlauch lediglich (lose) eingeschobene Spiralfeder mit radialem Übermaß gegenüber dem Innenschlauch bewirkt ein Aufweiten des gesamten Verbundschlauchs und hierbei ein Ausprägen der Federwindungen an der Außenseite des Außenschlauchs. Die Spiralfeder unterstützt hierbei die Widerlagerfunktion des Außenschlauchs und trägt somit dazu bei, dass der Endoskopschaft während seines Vortiebsmittels der Antriebsvorrichtung im Bereich der Antriebsmittel nicht gequetscht oder radial deformiert wird. Die Spiralfeder erhöht dabei unwesentlich die Biegeflexibilität des Endoskopschafts über dessen gesamte Länge. Schließlich bewirkten die spiralförmig umlaufenden Rillen an der Außenseite des Außenschlauchs, verursacht durch die radialen Anpresskräfte der Spiralfeder, eine Verringerung der Anlagefläche zwischen dem Endoskopschaft und einem dem Endoskopschaft umgebenden Antriebsstülpschlauch. Aufgrund dieser verringerten Auflagefläche können Reibkräfte zwischen Endoskopschaft und Stülpschlauch reduziert werden. Schließlich bilden die Rillen Schmiermitteltaschen, in denen ein zwischen den Endoskopschaft und den Antriebsstülpschlauch gepresstes Schmiermittel eingelagert wird.

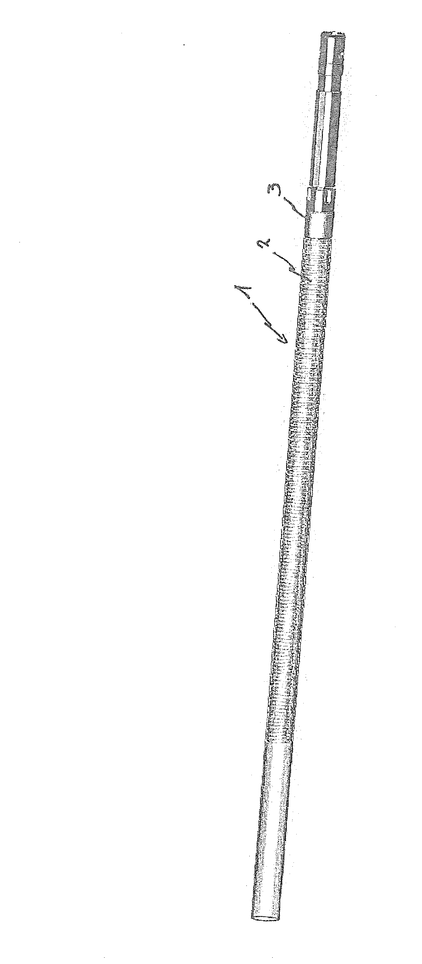

## Patentansprüche

1. Endoskopschaft aus einem Verbundschlauch (1) mit einem Außenschlauch und einem zum Außenschlauch weicheren und/oder biegeflexibleren Innenschlauch sowie einer Spiralfeder (2) zur Aussteifung des Verbundschlauchs (1), **dadurch gekennzeichnet, dass** die Spiralfeder (2) in entspanntem Zustand einen Außendurchmesser hat, der größer ist als der Innendurchmesser des Innenschlauchs und die Spiralfeder (2) in den Innenschlauch vorzugsweise lose unter einer Federvorspannung eingesetzt ist, wodurch sie sich gegen den Innenschlauch radial andrückt und dass weiterhin die radiale Andrückkraft so groß gewählt ist, dass die Spiralfeder (2) durch die Wandungen des Innenschlauchs und Außenschlauchs hindurch eine Struktur auf der Außenseite des Außenschlauchs aufprägt.

2. Endoskopschaft nach Anspruch 1, **dadurch gekennzeichnet, dass** die Struktur auf den Außenseite des Außenschlauchs den Federwindungen der Spiralfeder (2) entspricht und vorzugsweise spiralförmige Erhebungen und Vertiefungen bildet.

3. Endoskopschaft nach einem der vorstehenden Ansprüche **dadurch gekennzeichnet, dass** der Außenschlauch 92 Shore A und der Innenschlauch 60 Shore A hat.

4. Endoskopschaft nach Anspruch 3, **dadurch gekennzeichnet, dass** der Außenschlauch aus PUR und der Innenschlauch aus PVC besteht.

5. Endoskopschaft nach einem der vorstehenden Ansprüche, **dadurch gekennzeichnet, dass** die Außen- und Innenschlauch co-extrudiert sind.

6. Endoskopschaft nach einem der vorstehenden Ansprüche, **dadurch gekennzeichnet, dass** die Wandstärke des Außenschlauchs kleiner ist als die des Innenschlauchs.

7. Endoskopschaft nach einem der vorstehenden Ansprüche, **dadurch gekennzeichnet, dass** die Spiralfeder (2) eine rechtwinklige Federgeometrie hat.

8. Endoskopschaft nach einem der vorstehenden Ansprüche, **dadurch gekennzeichnet, dass** die Schaftlänge ca. 2.5 m beträgt.

9. Endoskop mit einem Stülpschlauchantrieb bestehend aus einem vorzugsweise doppelt gestülpen Antriebsschlauch, der von einer Antriebseinrichtung umgriffen ist mit Antriebsmitteln, die in Reibeingriff mit dem Antriebsschlauch stehen, um eine Vorschubkraft auf den Antriebsschlauch aufzubringen, **gekennzeichnet durch** einen Endoskopschaft gemäß einem der Ansprüche 1 bis 8, der in dem Antriebsschlauch gleitend geführt ist und der ein radiales Widerlager für die Antriebsmittel bildet.

## Claims

1. An endoscope shaft made of a composite tube (1) comprising an external tube and an internal tube softer and/or more resilient compared to the external tube as well as a helical spring (2) for stiffening the composite tube (1), **characterized in that** the helical spring (2) in the relaxed state has an outer diameter which is larger than the inner diameter of the internal tube and the helical spring (2) is inserted in the internal tube preferably loosely at a spring bias, whereby it is radially pressed against the internal tube, and that furthermore the radial pressing force is chosen to be so high that the helical spring (2) impresses a structure onto the outside of the external tube through the walls of the internal tube and the external tube.

2. The endoscope shaft according to claim 1, **characterized in that** the structure on the outside of the external tube corresponds to the spring windings of the spiral spring (2) and forms preferably helical elevations and indentations.

3. The endoscope shaft according to any one of the preceding claims, **characterized in that** the external tube has 92 Shore A and the internal tube has 60 Shore A.

4. The endoscope shaft according to claim 3, **characterized in that** the external tube consists of PUR and the internal tube consists of PVC.

5. The endoscope shaft according to any one of the preceding claims, **characterized in that** the external and internal tubes are co-extruded.

6. The endoscope shaft according to any one of the preceding claims, **characterized in that** the wall thickness of the external tube is smaller than that of the internal tube.

7. The endoscope shaft according to any one of the preceding claims, **characterized in that** the helical spring (2) exhibits rectangular spring geometry.

8. The endoscope shaft according to any one of the preceding claims, **characterized in that** the shaft length is approx. 2.5 m.

9. An endoscope comprising an everting tube drive consisting of a drive tube preferably everted twice which is encompassed by a drive device including drive means which are in frictional engagement with the drive tube so as to apply a feed force to the drive tube, **characterized by** an endoscope shaft according to any one of claims 1 to 8 which is guided to slide in the drive tube and which forms a radial counter bearing for the drive means.

## Revendications

1. Tube d'endoscope réalisé dans un tube flexible composite (1) comportant un tube flexible extérieur et un tube flexible intérieur plus souple et/ou plus flexible que le tube extérieur, ainsi qu'un ressort à boudin (2) destiné à rigidifier le tube flexible composite (1), **caractérisé en ce que** le ressort à boudin (2) à l'état détendu a un diamètre extérieur supérieur au diamètre intérieur du tube flexible intérieur, et ledit ressort à boudin (2) est inséré dans le tube flexible intérieur, de préférence de manière lâche sous l'effet d'une précontrainte du ressort, moyennant quoi il vient se presser radialement contre le tube flexible intérieur, et **en ce que**, en outre, la force de pression radiale est choisie avec une valeur telle que le ressort à boudin (2) imprime au travers des parois du tube flexible intérieur et du tube flexible extérieur une structure sur la face extérieure du tube flexible extérieur.

2. Tube d'endoscope selon la revendication 1, **caractérisé en ce que** la structure sur la face extérieure du tube flexible extérieur correspond aux spires du ressort à boudin (2) et forme de préférence des bosses et des creux en forme de spirale.

3. Tube d'endoscope selon l'une des revendications précédentes, **caractérisé en ce que** le tube flexible extérieur a une dureté de 92 Shore A et le tube flexible intérieur a une dureté de 60 Shore A.

4. Tube d'endoscope selon la revendication 3, **caractérisé en ce que** le tube flexible extérieur est réalisé en PUR et le tube flexible intérieur en PVC.

5. Tube d'endoscope selon l'une quelconque des revendications précédentes, **caractérisé en ce que** le tube flexible extérieur et le tube flexible intérieur sont co-extrudés.

6. Tube d'endoscope selon l'une quelconque des revendications précédentes, **caractérisé en ce que** le tube flexible extérieur a une épaisseur de paroi inférieure à celle du tube flexible intérieur.

7. Tube d'endoscope selon l'une quelconque des revendications précédentes, **caractérisé en ce que** le ressort à boudin (2) a une géométrie de ressort rectangulaire.

8. Tube d'endoscope selon l'une quelconque des revendications précédentes, **caractérisé en ce que** ledit tube a une longueur de 2,5 m environ.

9. Endoscope comportant un système de commande à tube flexible retroussé, constitué d'un tube flexible de commande de préférence à double retroussement, qui est entouré d'un dispositif de commande avec des moyens de commande, qui sont en prise par frottement avec le tube flexible de commande afin d'appliquer une force d'avancement sur le tube flexible de commande, **caractérisé par** un tube d'endoscope selon l'une quelconque des revendications 1 à 8, qui est logé de manière à glisser dans le tube flexible de commande et qui forme une butée radiale pour les moyens de commande.
